# EUROPEAN PATENT SPECIFICATION

(11) **EP 1 196 366 B1**
(45) Date of publication and mention of the grant of the patent: **20.08.2003**
(21) Application number: 00946210.2
(22) Date of filing: 24.07.2000
(51) Int. Cl.: C07C 51/12, C07C 51/14, C07C 53/08

(54) **CARBONYLATION REACTIONS**
CARBONYLIERUNGSREAKTIONEN
REACTIONS DE CARBONYLATION

(30) Priority: 23.07.1999 GB 9917429
(43) Date of publication of application: 17.04.2002
(73) Proprietor: THOMAS SWAN AND CO., LTD., Consett, Co. Durham DH8 7ND (GB)
(72) Inventor: COLE-HAMILTON, David, John, St. Andrews, Fife KY16 9ST (GB); SELLIN, Murielle, F., Fife KY16 9ST (GB)
(74) Representative: Atkinson, Jonathan David Mark
(86) International application number: GB0002847
(87) International publication number: WO01007388

(56) References cited:
- WO-A-00/01651
- WO-A-97/38955
- GB-A- 2 100 729
- US-A- 5 198 589

## Description

The present invention relates to a method for carrying out carbonylation reactions.

The use of carbon monoxide as a reagent for organic synthesis is diverse with a wide number of reactions carried out. The carbonylation of methanol is an important industrial process producing millions of tons of acetic acid per year. The preferred process, that of BP-Monsanto using cobalt or rhodium based homogeneous catalysts, is a continuous batch process which requires distillation to separate catalyst and products.

In general, carbonylation reactions involve reaction of an alkene or alcohol with carbon monoxide over a catalyst at high pressure to produce a carbonyl compound.

It is well known that reactions of this type are limited by the solubility of the gases in the liquid reagent or solvent (known as Mass Transport Limitations).

A further problem is that the use of homogeneous catalysts requires a separation step at the end of the process to recover the catalyst. This is particularly difficult in the case of alkenes/alcohols longer than C7 where separating the catalysts from the products by distillation requires high temperatures which may destroy the catalysts. This results in it being impossible for these processes to be carried out in continuous flow reactors (tubular reactors).

The use of homogeneous catalysts means that these processes are usually carried out in batch or semi-batch reactors requiring extensive capital expenditure when scaling up due to the need to design vessels capable of working at high pressure. The use of batch systems also gives increased down time for charging and discharging as well as yielding a product which will be a mixture of thermodynamic and kinetic products due to the large residence time in the reactor.

Heterogeneous catalysed carbonylation of methanol has been attempted in both liquid and gas phases. The liquid phase routes suffer from leaching out of the catalyst and the active species, the catalyst then acting in a homogenous manner, thus defeating the whole point of the supported catalyst. It is known that the rate of carbonylation by the homogenous route is at least twice that of the heterogeneous route. The gas phase reaction with a support catalyst has the disadvantage that only low substrate concentrations are achievable in the gas phase, thus limiting reaction rate. Gas phase reactions, because of the higher temperatures employed suffer from side-reactions.

WO97/38955 discloses a process for the selective hydrogenation of aliphatic or aromatic substrates under supercritical or near-critical conditions. The conditions of the process are controlled so as to ensure selective hydrogenation of a specified carbon-carbon double bond without removing the unsaturation of other carbon-carbon double bonds which are also present.

GB2100729 discloses a method of producing acyl fluorides by reacting carbon monoxide, anhydrous hydrogen fluoride and an organic compound, such as propylene, under supercritical conditions.

US5198589 discloses a method of olefin hydroformulation in which a olefin is reacted with hydrogen and carbon monoxide in the presence of a cobalt carbonyl catalyst.

### The process provides higher yields of the n-isomer product

The use of supercritical fluids to replace conventional solvents for environmental reasons is gradually being realised. However the use of supercritical fluids also gives higher solubility of reagents in the system and gives effectively a higher activity to these reagents by overcoming Mass Transport limitations.

For this reason work has already been carried out on batch systems using homogeneous catalysts in supercritical fluids e.g.
1 Carbonylation of aryl halides catalysed by palladium complexes with phosphite ligands in supercritical carbon dioxide
   Kayaki, Yoshihito; Noguchi, Yushi; Iwasa, Seiji; Ikariya, Takao; Noyori, Ryoji Chem. Commun. (1999), (13), 1235-1236
2 Catalytic carbonylations in supercritical carbon dioxide
   Ojima, Iwao; Tzamarioudaki; Maria; Chuang, Chih-Yuan; lula, Donna M.; Li, Zhaoyang
   Chem. Ind. (Dekker) (1998), 75 (Catalysis of Organic Reactions), 333-343

According to the present invention, there is provided a process for the carbonylation of a substrate selected from alkenes, alkynes and alcohols wherein the substrate is reacted with carbon monoxide over a heterogeneous metal catalyst with or without an additional solvent and wherein at least one component selected from the group comprising: carbon dioxide, an alkene, an alkyne, a hydrocarbon, a halocarbon and nitrogen, or one of the reactants, or a mixture of two or more of these is under supercritical or near-critical conditions.

Near critical is defined herein as being a set of conditions below the critical temperature and pressure where the density of the fluid is sufficient to give high enough solubility of the starting material in the system to carry out the carbonylation.

We have found that it is possible, by using a combination of supercritical fluids and a heterogeneous catalyst, in particular a supported metal catalyst where the metal is preferably ruthenium, rhodium, platinum or cobalt in the form of a complex, optionally with a promoter such as methyl iodide, to carry out carbonylation with high conversion, good selectivity and reduced catalyst leaching. Surprisingly it was found that catalyst leaching for polyvinylpyrrolidone supported catalysts was much reduced compared to the liquid phase system. In addition to being utilised bound to the catalyst, as is particularly possible with methyl iodide, the promoter may also be included in the feed to the reactor.

Not only is this considered to be the first heterogeneous (supported catalyst) catalysed carbonylation carried out in a supercritical fluid to be reported but this gives significant industrial advantage as catalysts can be easily separated from product by simple filtration even for carbonylation of alcohols, as well as alkenes or alkynes, of chain lengths greater than C7.

The use of a heterogeneous/supported catalyst in turn allows the advantage of using a continuous flow reactor, typically a tubular reactor, where the ability to control residence time, as well as the other reaction parameters independently, allows greater control resulting in more efficient and also more selective process. This selectivity is important as other products can be formed in methanol carbonylation e.g. methyl acetate and dimethoxy ethane. The use of a tubular reactor also gives the advantage of having a low inventory of reagents under high pressure at any moment hence increasing the safety of the process and also ease of catalyst separation.

When carrying out the process of this invention, the supercritical fluid is carbon dioxide, propane, an alkene, an alkyne, ammonia, hydrocarbon, halocarbon, nitrogen, or one of the reactants, or a mixture of two or more of these.

Surprisingly we have found that under such conditions the reaction can be made very selective. A preferred process embodying this invention this comprises controlling one or more of the reaction conditions of temperature, pressure, residence time, flow rate and catalyst so as to enhance yield and/or selectivity of reaction. For example, alteration of reaction pressure gives selectivity with regards to the production of the acid or the ester (Table 1), Notably, methanol carbonylation can be accomplished with minimal catalyst leaching and then the products easily separated from the catalyst without distillation or further work-up.

### The following example illustrates this invention:

**Example:** The supported catalyst (rhodium on polyvinylpyrollidone) was loaded into an autoclave. Degassed methanol and methyl iodide were injected in against a stream of carbon dioxide, the autoclave was sealed, pressurised to 40 bar with carbon monoxide and then pressurised to reaction pressure with carbon dioxide. The amount of carbon dioxide was varied to control the reaction pressure on heating. At the end of the reaction the products were collected by cooling the autoclave to -80°C and venting. For results under various conditions see Table 1.

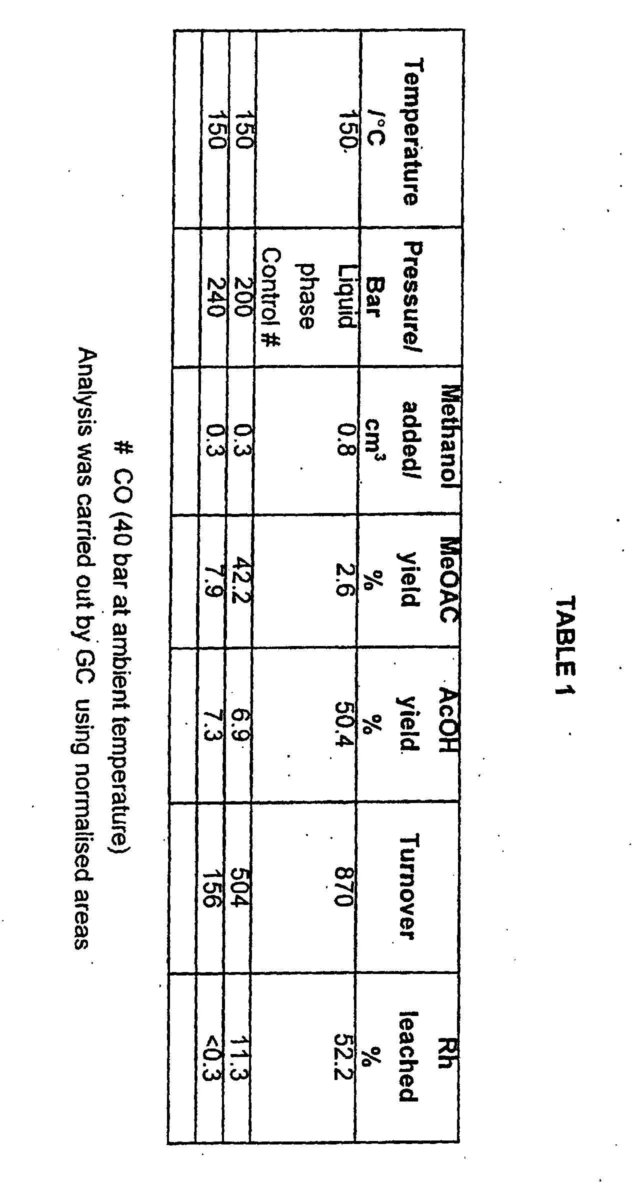

## Claims

1. A process for the carbonylation of a substrate selected from alkenes, alkynes, and alcohols wherein the substrate is reacted with carbon monoxide over a heterogeneous metal catalyst with or without an additional solvent and where at least one component selected from the group comprising: carbon dioxide, an alkene, an alkyne, a hydrocarbon, a halocarbon and nitrogen, or one of the reactants, or a mixture of two or more of these is under supercritical or near-critical conditions.

2. A process according to claim 1, wherein the reaction is carried out in a continuous flow reactor.

3. A process according to claim 1 or 2, wherein the substrate is an alcohol, alkene or alkyne containing more than seven carbon atoms.

4. A process according to any proceeding claim, wherein the catalyst is a supported metal catalyst.

5. A process according to any preceding claim, which is carried out in the presence of a promoter.

6. A process according to any one of claims 1 to 4, wherein the metal catalyst is a supported metal complex selected from metal complexes of platinum, rhodium, cobalt and ruthenium, the catalyst optionally having a promoter bound thereto.

7. A process according to claim 5 or 6 wherein the promoter is methyl iodide

## Patentansprüche

1. Verfahren zur Carbonylierung eines Substrats, ausgewählt aus Alkenen, Alkynen und Alkoholen, bei dem das Substrat über einem heterogenen Metallkatalysator mit oder ohne zusätzliches Lösungsmittel mit Kohlenmonoxid umgesetzt wird, wobei mindestens eine aus der Kohlendioxid, ein Alken, ein Alkyn, einen Kohlenwasserstoff, einen Halogenkohlenstoff und Stickstoff umfassenden Gruppe ausgewählte Komponente oder einer der Reaktanten oder ein Gemisch aus zwei oder mehreren von diesen in einem superkritischen oder nahezu kritischen Zustand ist.

2. Verfahren nach Anspruch 1, bei dem die Reaktion in einem Durchlaufreaktor durchgeführt wird.

3. Verfahren nach Anspruch 1 oder 2, bei dem das Substrat ein Alkohol, Alken oder Alkyn ist, das mehr als sieben Kohlenstoffatome aufweist.

4. Verfahren nach einem der vorstehenden Ansprüche, bei dem der Katalysator ein geträgerter Metallkatalysator ist.

5. Verfahren nach einem der vorstehenden Ansprüche, das in Gegenwart eines Beschleunigers durchgeführt wird.

6. Verfahren nach einem der Ansprüche 1 bis 4, bei dem der Metallkatalysator ein aus Metallkomplexen von Platin, Rhodium, Cobalt und Ruthenium ausgewählter geträgerter Metallkomplex ist, wobei ggfs. ein Beschleuniger an den Katalysator gebunden ist.

7. Verfahren nach Anspruch 5 oder 6, bei dem der Beschleuniger Methyliodid ist.

## Revendications

1. Procédé pour la carbonylation d'un substrat choisi entre des alcènes, des alcynes et des alcools, dans lequel le substrat est amené à réagir avec du monoxyde de carbone sur un catalyseur métallique hétérogène avec ou sans solvant supplémentaire, et dans lequel au moins un constituant choisi dans le groupe comprenant : le dioxyde de carbone, un alcène, un alcyne, un hydrocarbure, un hydrocarbure halogéné et l'azote, ou un des corps réactionnels, ou bien un mélange de deux ou plus de deux de ces composés est dans des conditions surcritiques ou pratiquement critiques.

2. Procédé suivant la revendication 1, dans lequel la réaction est conduite dans un réacteur à écoulement continu.

3. Procédé suivant la revendication 1 ou 2, dans lequel le substrat est un alcool, un alcène ou un alcyne contenant plus de 7 atomes de carbone.

4. Procédé suivant l'une quelconque des revendications précédentes, dans lequel le catalyseur est un catalyseur métallique fixé sur un support.

5. Procédé suivant l'une quelconque des revendications précédentes, qui est mis en oeuvre en présence d'un promoteur.

6. Procédé suivant l'une quelconque des revendications 1 à 4, dans lequel le catalyseur métallique est un complexe métallique fixé sur un support, choisi parmi des complexes métalliques de platine, de rhodium, de cobalt et de ruthénium, le catalyseur comprenant facultativement un promoteur lié à celui-ci.

7. Procédé suivant la revendication 5 ou 6, dans lequel le promoteur est l'iodure de méthyle.
